# EUROPEAN PATENT APPLICATION

(11) **EP 4 112 603 A1**
(43) Date of publication of application: **04.01.2023**
(21) Application number: 21382571.4
(22) Date of filing: 29.06.2021
(51) Int. Cl.: C07D 233/86, C07C 237/00

(54) **PROCESSES FOR THE PREPARATION OF NON-STEROIDAL ANTIANDROGENS**

(71) Applicant: Química Sintética, S.A., 28805 Alcalá de Henares (ES)
(72) Inventor: BARRECA, Giuseppe, SIRTORI, LC. (IT); LIMING, Huang, HANGZHOU CITY (CN)
(74) Representative: Ponti & Partners, S.L.P

(57) **Abstract**

An improved preparation of non-steroidal antiandrogen drugs, such as 4-[3-[4-cyano-3-(trifluoromethyl)phenyl]-5,5-dimethyl-4-oxo-2-thioxo-1-imidazolidinyl]-2-fluoro-N-methyl-benzamide and intermediates thereof, is described.

## Description

### Field of the invention

The present invention relates to the preparation of non-steroidal antiandrogen drugs, such as 4-[3-[4-cyano-3-(trifluoromethyl)phenyl]-5,5-dimethyl-4-oxo-2-thioxo-1-imidazolidinyl]-2-fluoro-N-methyl-benzamide and intermediates thereof.

### Background

4-[3-[4-Cyano-3-(trifluoromethyl)phenyl]-5,5-dimethyl-4-oxo-2-thioxo-1-imidazolidinyl]-2-fluoro-N-methyl-benzamide (enzalutamide, marketed under the brand name Xtandi^{®}), is a nonsteroidal antiandrogen (NSAA) medication indicated for use in the treatment of metastatic castration-resistant prostate cancer (mCRPC) and non-metastatic castration-resistant prostate cancer. It is taken by oral administration.

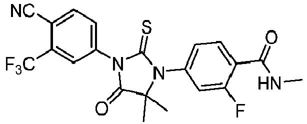

Enzalutamide was first described in 2006, and was introduced for the treatment of prostate cancer in 2012. It was the first NSAA of second generation to enter the market. The medication is widely available throughout the world.

Enzalutamide was approved by the U.S. Food and Drug Administration (FDA) on August 31, 2012, then approved by European Medicine Agency (EMA) on June 21, 2013, and approved by Pharmaceuticals and Medical Devices Agency of Japan (PMDA) on March 24, 2014.

A first route of synthesis for enzalutamide was disclosed in WO 2006/124118. Due to the scarce selectivity of the last coupling step, this synthetic approach was deemed unsuitable for industrial production purposes.

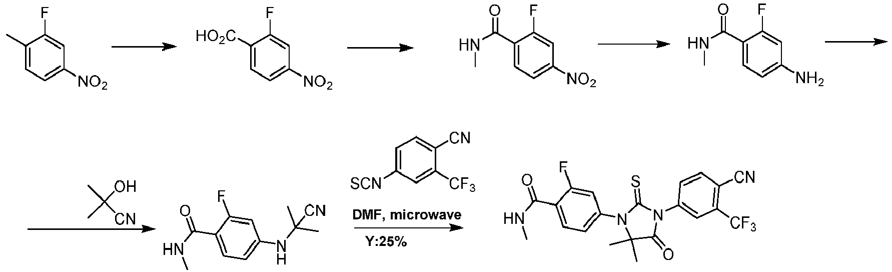

Numerous synthetic strategies have been proposed over years for the preparation of enzalutamide, mostly entailing a convergent approach. Of particular relevance is that described in WO 2011/106570, wherein the thiohydantoin ring can be prepared through the coupling of two key fragments, referred to in the following as fragment A and fragment B.

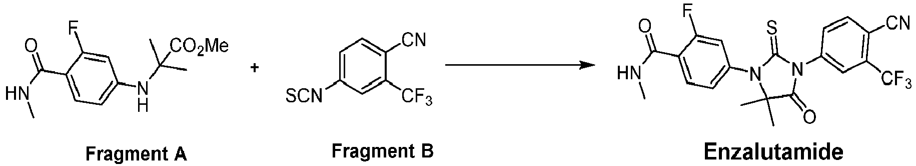

It was experimentally found that the WO 2011/106570 procedure suffers of significant disadvantages concerning low yield with respect to fragment B, which impacts on the overall cost of the process, and purity of the final product.

WO 2015/154730 discloses a process wherein the coupling between fragment A-acid and fragment B (as shown above) is carried out in the presence of a large excess of phenol (up to 6 equivalents with respect to fragment A-acid), in order to minimize the formation of the oxo-enzalutamide impurity indicated as "oxo-enzalutamide".

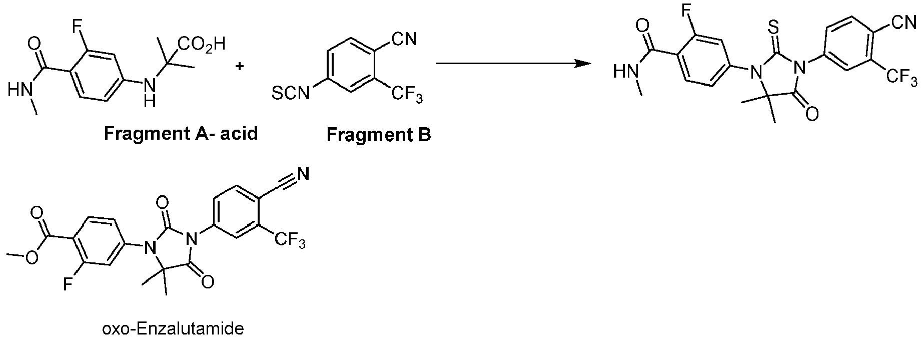

A significant drawback of this method is linked to the use of large quantities of phenol, which is corrosive, highly irritating and toxic to humans, hence this method entails safety issues, which make it unsuitable for large scale production.

An object of the present invention is, therefore, to provide a synthetic approach to the preparation of enzalutamide, and related structures, that is cost-effective, with improved safety and feasible on large scale, minimizing or avoiding the formation of impurities deriving from side-reactions.

### Summary of the invention

These objectives, together with other objectives which will be made evident hereafter, are achieved by the present invention that, in an aspect thereof, relates to a process for the preparation of a compound having the structure of formula (I) 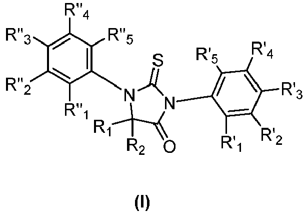 wherein:
- R₁ and R₂, each independently from the other, can be H, C₁-C₈-alkyl or C₆-C₁₀-aryl, optionally substituted with one or more halide, cyano, hydroxy or amino group(s);
- one or more of R'₁ - R'₅ and of R"₁ - R"₅, each independently from the others, is selected from the group consisting of R₁, cyano, -COOR₁, -CONR₁R₂, -OCOR₁, - OCNR₁R₂, wherein R₁ and R₂ are as defined above,
- the process comprising the step of coupling the compound of formula (II) and the compound of formula (III) to obtain the compound of formula (I) 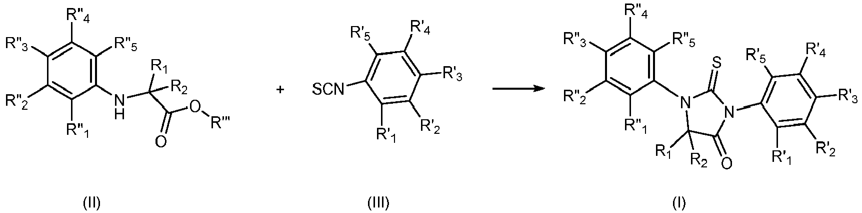
- wherein R'" is a phenyl bearing one or more substituent(s) independently selected from halide, -NO₂, -OR₁, C₂-C₈-alkyl, cyano, COOR₁, -CONR₁R₂, -OCOR₁, - OCNR₁R₂, wherein R₁ and R₂ are as defined above, or wherein R''' is a biphenyl or a naphthyl, optionally bearing one or more substituent(s) independently selected from halide, -NO₂, -OR₁, R₁, cyano, -COOR₁, -CONR₁R₂, -OCOR₁, -OCNR₁R₂, wherein R₁ and R₂ are as defined above.

The meaning of R'₁ - R'₅ and of R"₁ - R"₅ defined above are applicable indistinctly in formulae (I), (II) and (III), or a derivate thereof.

In another aspect, the present invention relates to the compound of general formula (IIa) 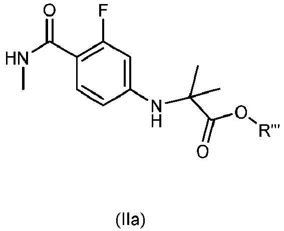 wherein R''' is a phenyl bearing one or more substituent(s) independently selected from halide, -NO₂, -OR₁, C₂-C₈-alkyl, cyano, COOR₁, -CONR₁R₂, -OCOR₁, -OCNR₁R₂, wherein R₁ and R₂ are as defined above, or wherein R''' is a biphenyl or a naphthyl optionally bearing one or more substituent(s) independently selected from halide, NO₂, -OR₁, R₁, cyano, - COOR₁, -CONR₁R₂, -OCOR₁, -OCNR₁R₂, wherein R₁ and R₂ are as defined above.

Preferably, R''' is para-nitrophenyl named herein compound (IIb) or 4-chlorophenyl named herein compound (IIc).

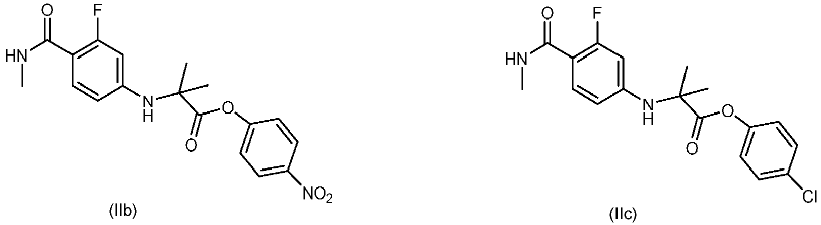

In another aspect, the present invention relates to a process for the preparation of the compound of general formula (IIa) as described above, comprising the step of esterification of the acid of formula (IV) with a phenol derivative or hydroxydiphenyl or naphthol, or a derivative thereof, of formula R'''OH, wherein R''' is as defined above, preferably wherein R'" is para-nitrophenyl.

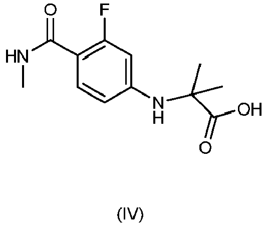

In an aspect, the present invention relates to the use of a compound of general formula (IIa) or compound of formula (IIb) or compound of formula (IIc) for the preparation of enzalutamide.

In another aspect, the present invention relates to a pharmaceutical formulation comprising 4-[3-[4-cyano-3-(trifluoromethyl)phenyl]-5,5-dimethyl-4-oxo-2-thioxo-1-imidazolidinyl] -2-fluoro-N-methyl-benzamide obtained via the process described above, optionally in admixture with at least one pharmaceutically acceptable excipient.

### Detailed description of the invention

All terms used in this application, unless otherwise specified, are to be understood in their ordinary meaning as known in the technical field.

The term "*about*" includes the range of experimental errors, which can normally occur performing a measurement, e.g. ± 5% or ±2% or ±1%.

The term "*mass*" defines the combination of substrates, reagents, solvents, and products on which a physical or chemical transformation is carried out.

The term "*excipient*" means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of giving a shape, to facilitate administration and preserve the active ingredient. Furthermore, they contribute to characterize the pharmaceutical preparation from the point of view of appearance, stability, biopharmaceutical profile and acceptability by the patient.

Unless otherwise indicated, in the context of the present invention the percentage and amount of a certain component in a composition are to be referred to the weight of said component with respect to the total weight of the composition.

Unless otherwise indicated, in the context of the present invention the indication that a composition "*comprises"* other one or more components/elements means that the indicated components/elements must be present and also other components may be present, but are not necessarily present, in the composition, in addition to the ones specifically recited. In other words, the indication that a composition "*comprises"* one or more components does not exclude that the composition *consists* of, or consists essentially of, the recited component(s). Similarly, the indication that a process "comprises" one or more steps does not exclude that the process comprises steps, such as synthetic steps or purification steps, in addition to the one or more steps explicitly recited.

As used herein, the indication that a compound or composition A is *"entirely free*" of other substances (or "*consists of*") means that, within the detection range of the instrument or method being used, no substances other than those specifically indicated can be detected in A.

Unless otherwise indicated, in the context of the present invention a range of values indicated for a certain parameter, for example the weight of a component in a mixture, includes the upper and the lower limits of the range, e.g. if the content in weight, or in volume, of a component A in a mixture is indicated as "*X to Y*", the content of A can be X, Y or any of the intermediate values.

In one aspect, the present invention relates to a process for the preparation of a compound having the structure of formula (I) 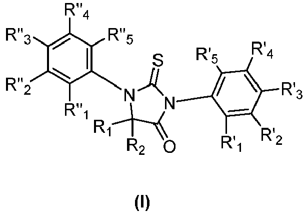 wherein:
- R₁ and R₂, each independently from the other, can be H, C₁-C₈ alkyl or aryl, optionally substituted with one or more halide, cyano, hydroxy or amino group(s);
- one or more of R'₁ - R'₅ and of R"₁ - R"₅, each independently from the others, is selected from the group consisting of R₁, cyano, halide, -COOR₁, -CONR₁R₂, - OCOR₁, -OCNR₁R₂, wherein R₁ and R₂ are as defined above,
- the process comprising the step of coupling the compound of formula (II) and the compound of formula (III) to obtain the compound of formula (I) 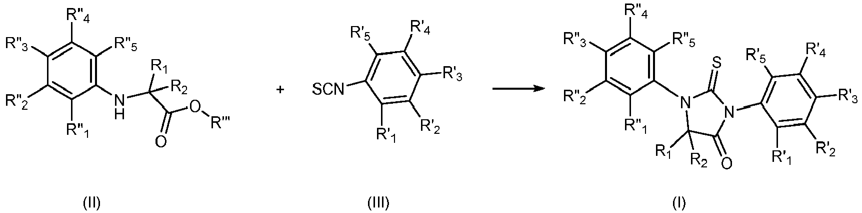
- wherein R''' is a phenyl bearing one or more substituent(s) independently selected from halide, -NO₂, -OR₁, C₂-C₈-alkyl, cyano, COOR₁, -CONR₁R₂, -OCOR₁, - OCNR₁R₂, wherein R₁ and R₂ are as defined above, or wherein R'" is a biphenyl or a naphthyl optionally bearing one or more substituent(s) independently selected from halide, -NO₂, -OR₁, R₁, cyano, -COOR₁, -CONR₁R₂, -OCOR₁, -OCNR₁R₂, wherein R₁ and R₂ are as defined above.

The above process is suitable to be used for the preparation of 4-[3-[4-cyano-3-(trifluoromethyl)phenyl]-5,5-dimethyl-4-oxo-2-thioxo-1-imidazolidinyl]-2-fluoro-N-methyl-benzamide (enzalutamide) and of several analogues thereof.

Upon testing the known approaches, in particular that of WO 2011/106570, it was found that the coupling of fragments A and B requires at least 2 equivalents of the latter (namely the one bearing the isothiocyanate moiety). Without intending to be bound by theory, this is likely due to the tendency of the methanol, produced during the course of the reaction, to react with fragment B itself. This results in the formation of the corresponding methyl carbamate, an impurity which must be removed from the reaction product, and ultimately subtracts fragment B from the reaction mixture. It was observed that, due to the long times required for the reaction and the partial instability of fragment B under the reaction conditions used, at least 2.5 equivalents of this compound must be used to achieve a complete conversion of fragment A into the target molecule.

Being fragment B the reagent which most significantly impacts on the overall cost of the process, also for the use for its preparation of thiophosgene (a highly toxic compound requiring specific equipment for its production and manipulation) or a derivative thereof, the overall cost and feasibility of the WO 2011/106570 procedure was found to be far from ideal. The strategy involving the coupling of fragment B with the free acid form of fragment A (i.e. des-methyl fragment A following the approach of WO 2015/154730) was initially investigated. This approach was found to be scarcely selective, both on small and on a multigram scale, in that it leads to the formation, to a significant extent, of at least two impurities related to fragment A and very difficult to eliminate by crystallization.

Said impurities, referred to below as "*open-ring impurity*" (15-20% HPLC area with respect to Enzalutamide) and "*oxo-enzalutamide*" (2-3% HPLC area with respect to Enzalutamide), are shown in the following figure.

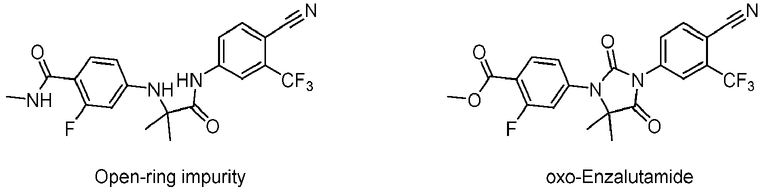

Accordingly, a multi-stage purification process was developed to reduce their content below the acceptance limit, thus resulting in an overall yield (referred to the preparation and purification of enzalutamide) of less than 60% with respect to fragment A.

Oxo-Enzalutamide turned out to be hardly purgeable by crystallization. Furthermore, any attempts to prevent its formation by modifying the reaction parameters failed. Its removal could be successfully achieved through crystallization from a methanol/water mixture (as described in Cryst. Growth. Des 2018, 18 (7), 3774-3780). It was, however, found that, while amounts of this impurity of about 2% can be effectively purged with a single crystallization procedure, the same crystallization conditions are not as effective in case of higher starting quantities, hence multiple crystallizations may be needed, with consequent loss of yield and diminished efficiency of the overall process.

It was further found that, although using des-methyl fragment A the consumption of the most expensive reagent (fragment B) has been significantly reduced with respect to the amounts required in WO 2011/106570 (1.5 vs 2.5 equivalents), the low selectivity of the reaction led to an overall yield and process cost not significantly differing from that of the process in WO 2011/106570.

The possibility of using an aromatic ester of fragment A in the coupling with fragment B was then investigated. Several aromatic esters were thus prepared using a series of phenols and aromatic compounds bearing an OH group, optionally substituted with electronwithdrawing or electron-donating groups, and tested in the coupling reaction with the fragment B. Particularly advantageous results, including complete conversion into the target molecule using as little as 1.3 equivalents of fragment B, were obtained with the following: phenol; 4-chlorophenol; 4-nitrophenol; pentafluorophenol; naphthol; 2,4-dichlorophenol; 4-tert-butyl phenol; and 4-methoxyphenol.

Preferably, in the process according to the present invention, at least one of R"₁-R"₅ is - CONHR₁, wherein R₁ is as defined in claim 1 or a fluorine group.

Preferably, in the process according to the present invention, at least one of R'₁-R'₅ is a trifluoromethyl or a cyano group.

Preferably, in the process according to the present invention, R''' is phenyl bearing one or more substituent(s) independently selected from halide, nitro, -OR₁, C₂-C₈-alkyl, cyano, - COOR₁, -CONR₁R₂, -OCOR₁, -OCNR₁R₂, R₁ and R₂ are as defined above.

The 4-nitrophenol ester of fragment A is particularly preferred in view of the fact that the corresponding phenol is widely commercially available and scarcely toxic and that the ester itself is a crystalline and easily purifiable solid.

The advantages of the synthesis according to the present invention were clearly shown in a series of experiments aimed at comparing it with the synthesis of WO 2011/106570.

Specifically, two sets of experiments, one relative to the synthesis of the present invention and one following the approach of WO 2011/106570, i.e. coupling using the methyl ester fragment A, were conducted, following the general coupling procedure in example 5 of WO 2011/106570 and varying the amount of fragment B with respect to fragment A with the aim of finding the minimum amount of fragment B necessary to convert completely fragment A into the final product. The purity profile, conversion and yield obtained with both synthetic approaches were evaluated.

For the procedure of WO 2011/106570 (coupling using the fragment A as methyl ester) it was confirmed that (all yields here below are titrated in the reaction mixture):
- Complete conversion of fragment A is achieved only when 2.5 equivalents of fragment B are used, with yield of about 95%.
- With 2 equivalents of fragment B (i.e. the amount reported in the example 5 of WO 2011/106570), the reaction stops at 95% conversion and the yield is about 91%.
- With lower amounts of fragment B the reaction does not proceed to complete conversion of fragment A via the procedure of WO 2011/106570.
- The methyl-carbamate impurity is formed since the beginning of the reaction. Its formation might explain the significant consumption of fragment B.
- Adding a large excess of fragment B, which would allow to convert completely fragment A into the final product, is the cause of the formation of a large amount of impurities related to fragment B degradation, which affect the purification of the final product.

The approach according to the present invention was carried out following the general procedure in example 5 of WO 2011/106570 and using, as non-limiting example, the 4-nitrophenol ester of fragment A (IIb) as shown hereunder:

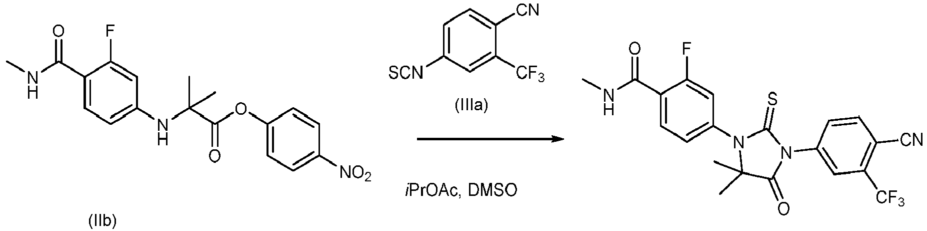

The following was observed:
Complete conversion of fragment A is achieved when from 1 to about 1.4 equivalents of fragment B (with respect to fragment A) are used and the corresponding yield resulted to be about 94%. Preferably, from 1.0 to 1.3 equivalents, more preferably from 1.1 to 1.2 equivalents, of fragment B with respect to fragment A are used.

With a slightly higher amount of fragment B, the reaction reaches the endpoint faster and only the impurities related to fragment B increase.

Thus, the synthetic route according to the present invention allows to produce enzalutamide with yield comparable to that of the process disclosed in WO 2011/106570 and using approximately half the amount of fragment B, consequently, lowering the amount of by products and improving the cost-effectiveness of the process.

In a preferred embodiment, the present invention relates to the process as described above, wherein at least one of R₁ and/or R₂ is selected from the group consisting of methyl, ethyl, propyl, isopropyl, fluorine, chlorine, bromine, iodine, trifluoromethyl and trichloromethyl.

In a preferred embodiment, the present invention relates to the process as described above, wherein compound (II) has the following formula (IIb)

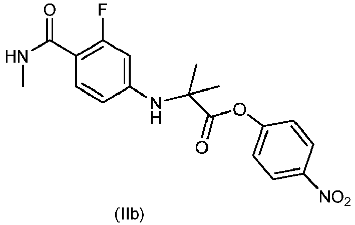

In another preferred embodiment combined or not with the previous embodiment, the present invention relates to the process as described above, wherein compound (III) has the following formula (IIIa):

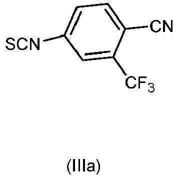

In a preferred embodiment, further comprising the step of preparing the compound of formula (II) by esterification of the corresponding carboxylic acid with phenol, hydroxydiphenyl, hydroxynaphthalene or a suitable derivative thereof, optionally in the presence of one or more condensation agent(s).

Among the known methods to prepare phenol esters, those involving the use of a coupling agent were found to ensure optimal selectivity and improved purity of the reaction mixture. Among the common coupling agents (included, i.e., carbonyl diimidazole), the best results were achieved using N,N'-dicyclohexylcarbodiimide (DCC) and 1-ethyl-3-(3-dimethylaminopropyl)carbodiimide hydrochloride (EDAC). Due to some issues related to the work-up (DCC forms an insoluble and hardly removable urea), EDAC is preferred as the coupling agent to be used since the corresponding urea shows high solubility in water (thus allowing for an easy removal thereof through aqueous work-up).

The coupling of compound of formula (IV) and a phenol derivative or hydroxydiphenyl or naphthol or a derivative thereof, in the presence of EDAC proceeds even in the absence of catalysts but the use of catalytic amounts of strong organic bases, e.g. 4-dimethylaminopyridine (DMAP), result in a significant increase of the reaction rate, limiting, at the same time, the formation of the corresponding N-acylureas (known by-products of this kind of reactions) thus increasing the yield.

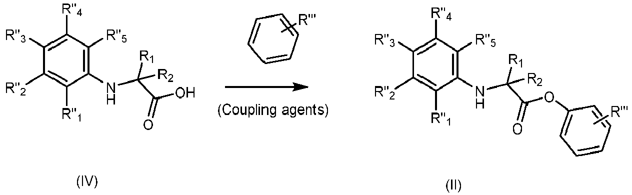

In an aspect, the present invention relates to a compound of general formula (IIa) 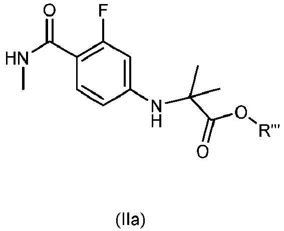 wherein R''' is a phenyl bearing one or more substituent(s) independently selected from halide, -NO₂, -OR1, C₂-C₈-alkyl, cyano, COOR₁, -CONR₁R₂, -OCOR₁, -OCNR₁R₂, or wherein R'" is a biphenyl or a naphthyl, optionally bearing one or more substituent(s) independently selected from halide, -NO₂, -OR₁, R₁, cyano, -COOR₁, -CONR₁R₂, -OCOR₁, -OCNR₁R₂, wherein R₁ and R₂ are as defined above.

Preferably, R''' is a phenyl bearing one substituent selected from the group consisting of - NO₂, pentafluoro, 2,4-dichloro or -difluoro, para-chloro, -bromo or -fluoro, linear or branched C₁-C₈ alkyl, linear or branched C₁-C₈ alkoxyl and a combination thereof.

More preferably, R''' is para-nitrophenyl, pentafluorophenyl, 4-chlorophenyl, 2,4-dichlorophenyl or 2,4-difluorophenyl. More preferably, in the compound of general formula (IIa) the R''' group is p-nitrophenyl.

In another aspect, the present invention relates to a process for the preparation of the compound of general formula (IIa) as described above, comprising the step of esterification of the acid of formula (IV) with a phenol derivative, hydroxydiphenyl or naphthol or a derivative thereof of formula R'''OH, wherein R'" is as defined above, preferably wherein R''' is para-nitrophenyl.

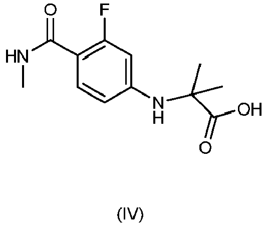

In an aspect, the present invention relates to the use of a compound of general formula (IIa) or compound of formula (IIb) or compound of formula (IIc) for the preparation of enzalutamide.

In another aspect, the present invention relates to a pharmaceutical formulation comprising 4-[3-[4-cyano-3-(trifluoromethyl)phenyl]-5,5-dimethyl-4-oxo-2-thioxo-1-imidazolidinyl] -2-fluoro-N-methyl-benzamide obtained via the process described above, optionally in admixture with at least one pharmaceutically acceptable excipient.

The following examples are provided to illustrate specific embodiments of the present invention, without intention to limit its scope.

### Example 1

### Preparation of 4-nitrophenyl 2-((3-fluoro-4-(methylcarbamoyl)phenyl)amino)-2-methyl propanoate (IIb).

In a 2-liter jacketed glass reactor equipped with mechanical stirrer, thermometer, reflux condenser and kept under nitrogen atmosphere are charged EDAC (1-ethyl-3-(3-dimethylaminopropyl)carbodiimide hydrochloride, 90 g), DMAP (N,N-dimethylaminopyridine, 3.4 g) and acetone (300 ml). The suspension is cooled under stirring to about -10°C.

In another 500-ml jacketed glass reactor equipped with mechanical stirrer, thermometer, reflux condenser and kept under nitrogen atmosphere are charged 2-((3-fluoro-4-(methylcarbamoyl)phenyl)amino)-2-methylpropanoic acid (100 g), 4-nitrophenol (60 g) and acetone (200 ml). The suspension is cooled under stirring to about 0°C and slowly transferred portion-wise, in about 15 minutes, into the reactor containing the suspension of EDAC, DMAP and acetone.

The reaction mixture is kept under stirring at -10/-5°C until reaction is complete.

When the reaction is over (HPLC), to the mixture maintained under stirring at 0-5°C, water (900 ml) is added dropwise, in not less than 30 minutes, while keeping internal temperature below 15°C. After about 50% of water addition, a white solid starts to precipitate.

The resulting suspension is kept under stirring at 0-5°C for at least 2 hours, filtered and reactor and the cake are washed with water.

Wet crude (IIb) (about 210 g) is used as it is for the following purification step.

Basing on the loss on drying the corresponding dry product corresponds to about 135 g (91% of the theoretical yield).

In a 2-liters jacketed glass reactor equipped with mechanical stirrer, thermometer, reflux condenser and kept under nitrogen atmosphere are charged 210 g of wet crude (IIb) (all the amount coming from the previous step), acetone (600 ml) and water (100 ml).

The suspension is heated under stirring to 50-55°C until almost all the solid is dissolved and a slight turbidity is present. Solution is filtered through a pad of celite and charcoal and combined with the previous filtered solution.

The clean solution of (IIb) is charged in the same reactor, and while keeping internal temperature at 50-55°C water (800 ml) is added dropwise, in not less the 30 minutes.

After about 50% of the addition precipitation of a white solid occurs. The suspension is cooled in about 2 hours to 0-5°C, kept under stirring in these conditions for not less than 2 hours and filtered. Reactor and the cake are washed with water.

The wet product (about 150 g) is dried under vacuum at 40-45°C until residual water (by KF analysis) is less than 0.2%. 125 g of (IIb, 85% yield) are obtained.

### Example 2: Preparation of 4-[3-[4-cyano-3-(trifluoromethyl)phenyl]-5,5-dimethyl-4-oxo-2-thioxo-1-imidazolidinyl] -2-fluoro-N-methyl-benzamide (Enzalutamide).

In a 2-liter jacketed glass reactor, equipped with mechanical stirrer, thermometer, condenser and kept under nitrogen atmosphere are charged compound of formula (IIIa) (4-isothiocyanato-2-(trifluoromethyl)benzonitrile, 106 g, obtainable as disclosed in WO 2011/106570), compound of formula (IIb) (125 g), isopropyl acetate (250 ml) and DMSO (125 ml).

The suspension is heated under stirring to 73-75°C and kept in these conditions for at least 24 hours before sampling for analysis. A solution is obtained.

When the reaction is complete (e.g. via HPLC analysis), the reaction solution is cooled to about 45-50°C, methanol (25 ml) is added and the mixture is kept in these conditions for 1 hour. While keeping internal temperature at 40-45°C isopropyl acetate (520 ml) and water (250 ml) are added.

The mixture is kept under stirring at 40-45°C for at least 15 minutes, the agitation is stopped and the layers are left to separate. The aqueous layer is discarded and the organic layer is washed at 40-45°C with water (250 ml).

The washed organic solution is concentrated under vacuum until a thick suspension is obtained. Methanol (250 ml) is added to the residue and the mixture is distilled in the same conditions until a thick residue is obtained.

Methanol (850 ml) is added to the residue and the mixture is heated under stirring to reflux until a solution is achieved and water (170 ml) is slowly added by maintaining internal temperature above 55°C.

The mixture is cooled to 40-45°C and kept under stirring in these conditions until abundant crystallization occurs (about 30 minutes), then stirred at 0-5°C and filtered.

The solid thus obtained can be further crystallized from isopropanol and water to obtain 115 g of dry enzalutamide as white crystals in the crystalline form R1 (75% molar yield from compound of formula (IIb)).

The procedure in examples 1 and 2 was repeated on a larger scale (starting from 200 g of 2-((3-fluoro-4-(methylcarbamoyl)phenyl)amino)-2-methylpropanoic acid) obtaining an 89% overall yield.

### Example 3: Preparation of 4-chlorophenyl 2-((3-fluoro-4-(methylcarbamoyl)phenyl)amino)-2-methyl propanoate, compound of formula (IIc).

In a 5-liter jacketed glass reactor equipped with mechanical stirrer, thermometer, reflux condenser and kept under nitrogen atmosphere are charged EDAC (211 g), DMAP (12.5 g) and acetone (1200 ml)

The suspension is cooled under stirring to about -5°C.

In another 1L jacketed glass reactor equipped with mechanical stirrer, thermometer, reflux condenser and kept under nitrogen atmosphere are charged 2-((3-fluoro-4-(methylcarbamoyl)phenyl)amino)-2-methylpropanoic acid (200 g), 4-chlorophenol (111.2 g) and acetone (750 ml). The suspension is cooled under stirring to about 0°C and slowly transferred portion-wise, in about 15 minutes, into the reactor containing the suspension of EDAC, DMAP and acetone.

The reaction mixture is kept under stirring at -10/-5°C until reaction is complete.

When the reaction is over (HPLC), to the mixture maintained under stirring at 0-5°C, water (2000 ml) is added dropwise, in not less than 30 minutes, while keeping internal temperature below 15°C. After about 50% of water addition, a white solid starts to precipitate.

The resulting suspension is kept under stirring at 0-5°C for at least 2 hours, filtered and reactor and the cake are washed with water.

The wet crude (about 400 g) is used as it is for the following purification step.

In a 5-liters jacketed glass reactor equipped with mechanical stirrer, thermometer, reflux condenser and kept under nitrogen atmosphere are charged 400 g of wet crude (from the previous step), acetone (600 ml) and water (100 ml).

The suspension is heated under stirring to 50-55°C until almost all the solid is dissolved and a slight turbidity is present. The solution is filtered through a pad of celite and charcoal and combined with the previous filtered solution.

The so-obtained clean solution is charged in the same reactor, and, while keeping internal temperature at 50-55°C water (1200 ml) is added dropwise, in not less the 30 minutes.

After about 50% of the addition precipitation of a white solid occurs. The suspension is cooled in about 2 hours to 0-5°C, kept under stirring in these conditions for not less than 2 hours and filtered. Reactor and the cake are washed with water.

The wet product (about 280 g) is dried under vacuum at 40-45°C. 237 g of the tile product are obtained (83% yield).

### Example 4: Preparation of 4-[3-[4-cyano-3-(trifluoromethyl)phenyl]-5,5-dimethyl-4-oxo-2-thioxo-1-imidazolidinyl] -2-fluoro-N-methyl-benzamide (Enzalutamide).

In a 5-liter jacketed glass reactor, equipped with mechanical stirrer, thermometer, condenser and kept under nitrogen atmosphere are charged compound of formula (IIIa) (4-isothiocyanato-2-(trifluoromethyl)benzonitrile,163 g, obtainable as disclosed in WO 2011/106570), 4-chlorophenyl 2-((3-fluoro-4-(methylcarbamoyl)phenyl)amino)-2-methyl propanoate (200 g), isopropyl acetate (400 ml) and DMSO (200 ml).

The suspension is heated under stirring to 60-70°C and kept in these conditions for at least 20 hours before sampling for analysis. A solution is obtained.

When the reaction is complete (e.g. via HPLC analysis), the reaction solution is cooled to about 45-50°C, methanol (30 ml) is added and the mixture is kept in these conditions for 1 hour. While keeping internal temperature at 40-45°C isopropyl acetate (1000 ml) and a 5% sodium carbonate solution in water (600 ml) are added.

The mixture is kept under stirring at 20-25°C for at least 15 minutes, the agitation is stopped and the layers are left to separate. The aqueous layer is discarded and the washed organic solution is concentrated under vacuum until a thick suspension is obtained. Methanol (400 ml) is added to the residue and the mixture is distilled in the same conditions until a thick residue is obtained.

Methanol (1400 ml) is added to the residue and the mixture is heated under stirring to reflux until a solution is achieved and a seed of crystalline enzalutamide (crystalline form R2) is added at 38-40°C and kept under stirring in these conditions until abundant crystallization occurs (about 30 minutes), then stirred at 0-5°C and filtered.

The solid thus obtained can be further crystallized from isopropanol and water to obtain 201 g of dry enzalutamide as white crystals in the crystalline form R1 (79% molar yield from 4-chlorophenyl 2-((3-fluoro-4-(methylcarbamoyl)phenyl)amino)-2-methyl propanoate).

### Example 5: The following intermediates of general formula (IIa) were prepared and used in the preparation of enzalutamide, with the overall yield for the preparation of enzalutamide (starting from 2-((3-fluoro-4-(methylcarbamoyl)phenyl)amino)-2-methylpropanoic acid) shown in the table below.

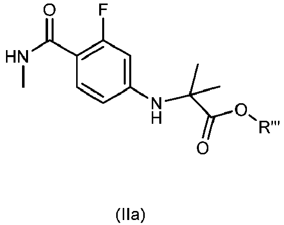

| **R''' is a phenyl bearing one substituent selected from the group consisting of:** | **Enzalutamide synthesis Overall yield%** |
|---|---|
| Penta-F | 88.2 |
| 2,4-di-Cl | 71.2 |
| 2,4-Di-F | 77.9 |
| 2-Cl | 73.7 |
| p-Br | 73.2 |
| Naphthol | 82.7 |
| p-F | 59.7 |
| p-tBu | 59.0 |
| p-MeO | 46.9 |

## Claims

1. A process for the preparation of a compound having the structure of formula (I) 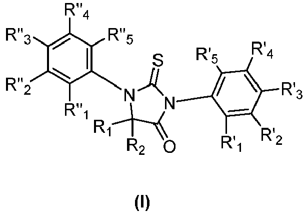 wherein:
- R₁ and R₂, each independently from the other, can be H, C₁-C₈-alkyl or C₆-C₁₀-aryl, optionally substituted with one or more halide, cyano, hydroxy or amino group(s);
- one or more of R'₁ - R'₅ and of R"₁ - R"₅, each independently from the others, is selected from the group consisting of R₁, cyano, halide, -COOR₁, -CONR₁R₂, - OCOR₁, -OCNR₁R₂, R₁ and R₂ being as defined above,
the process comprising the step of coupling the compound of formula (II) and the compound of formula (III) to obtain the compound of formula (I) 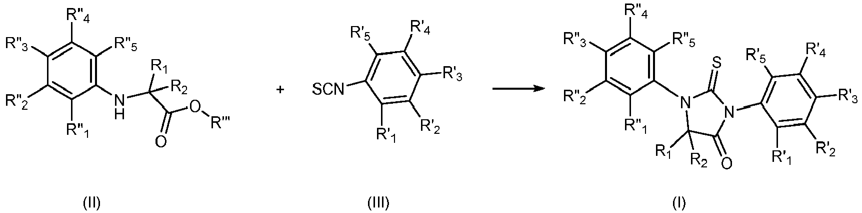 wherein R'" is phenyl bearing one or more substituent(s) independently selected from halide, -NO₂, -OR₁, C₂-C₈-alkyl, cyano, COOR₁, -CONR₁R₂, -OCOR₁, -OCNR₁R₂, wherein R₁ and R₂ are as defined above, or wherein R''' is a biphenyl or a naphthyl optionally bearing one or more substituent(s) independently selected from halide, -NO₂, -OR₁, R₁, cyano, -COOR₁, -CONR₁R₂, -OCOR₁, -OCNR₁R₂, wherein R₁ and R₂ are as defined above.

2. The process according to claim 1, wherein at least one of R₁ and/or R₂ is selected from the group consisting of methyl, ethyl, propyl, isopropyl, fluorine, chlorine, bromine, iodine, trifluoromethyl and trichloromethyl.

3. The process according to any of the preceding claims, wherein at least one of R"₁-R"₅ is -CONHR₁, wherein R₁ is as defined in claim 1 or a fluorine group.

4. The process according to any of the preceding claims, wherein at least one of R'₁-R'₅ is a trifluoromethyl or a cyano group.

5. The process according to any of the preceding claims, wherein R''' is phenyl substituted with one or more substituent(s) independently selected from halide, nitro, -OR₁, C₂-C₈-alkyl, cyano, -COOR₁, -CONR₁R₂, -OCOR₁, -OCNR₁R₂, R₁ and R₂ are as defined in claim 1.

6. The process according to any of the preceding claims, wherein compound (II) has the following general formula (IIa) 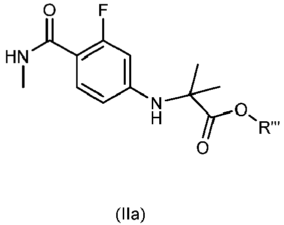 wherein R''' is a phenyl bearing one or more substituent(s) independently selected from halide, -NO₂, -OR₁, C₂-C₈-alkyl, cyano, COOR₁, -CONR₁R₂, -OCOR₁, -OCNR₁R₂, wherein R₁ and R₂ are as defined above, or wherein R''' is a biphenyl or a naphthyl optionally bearing one or more substituent(s) independently selected from halide, -NO₂, -OR₁, C₂-C₈-alkyl, cyano, -COOR₁, -CONR₁R₂, -OCOR₁, -OCNR₁R₂, wherein R₁ and R₂ are as defined in claim 1.

7. The process according to any of the preceding claims, wherein the step of coupling is performed with at least one of the followings: compound (II) has the following formula (IIb) 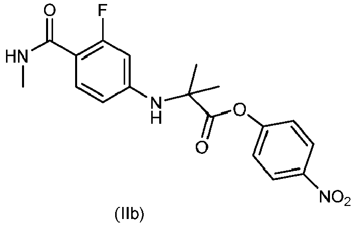 and compound (III) has the following formula (IIIa):

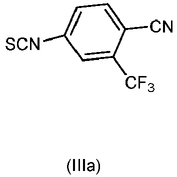

8. The process according to claim 7, wherein in the step of coupling the compound of formula (IIb) is coupled with the compound of formula (IIIa) and 4-[3-[4-cyano-3-(trifluoromethyl)phenyl]-5,5-dimethyl-4-oxo-2-thioxo-1-imidazolidinyl]-2-fluoro-N-methyl-benzamide is obtained and, optionally, purified via crystallization.

9. The process according to any of the preceding claims, further comprising the step of preparing the compound of formula (II) by esterification of the corresponding carboxylic acid with phenol, hydroxydiphenyl, hydroxynaphthalene or a suitable derivative thereof.

10. A compound of general formula (IIa) 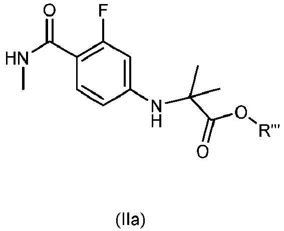 wherein R''' is a phenyl bearing one or more substituent(s) independently selected from halide, -NO₂, -OR1, C₂-C₈-alkyl, cyano, COOR₁, -CONR₁R₂, -OCOR₁, -OCNR₁R₂, or wherein R'" is a biphenyl or a naphthyl, optionally bearing one or more substituent(s) independently selected from halide, -NO₂, -OR₁, R₁, cyano, -COOR₁, -CONR₁R₂, -OCOR₁, -OCNR₁R₂, wherein R₁ and R₂ are as defined in claim 1.

11. The compound according to claim 10, wherein R''' is para-nitrophenyl, pentafluorophenyl, 4-chlorophenyl, 2,4-dichlorophenyl or 2,4-difluorophenyl, preferably wherein R'" is para-nitrophenyl, pentafluorophenyl or 4-chlorophenyl.

12. The compound according to claims 10-11 having the following formula (IIb)

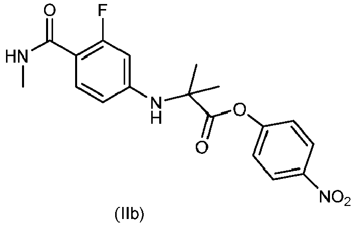

13. A process for the preparation of the compound of general formula (IIa) according to claim 10, comprising the step of esterification of the acid of formula (IV) with a phenol derivative or hydroxydiphenyl or naphthol, or a derivative thereof, of formula R'''OH, wherein R''' is as defined in claim 10, preferably wherein R'" is para-nitrophenyl.

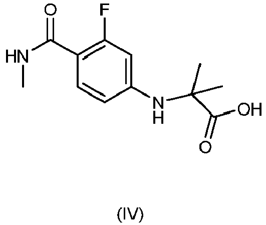

14. Use of a compound of general formula (IIa) or formula (IIb) for the preparation of enzalutamide.

15. A pharmaceutical formulation comprising 4-[3-[4-cyano-3-(trifluoromethyl)phenyl]-5,5-dimethyl-4-oxo-2-thioxo-1-imidazolidinyl]-2-fluoro-N-methyl-benzamide obtained according to the process of any of claims 1-9, optionally in admixture with at least one pharmaceutically acceptable excipient.
